# EUROPEAN PATENT APPLICATION

(11) **EP 1 148 064 A1**
(43) Date of publication of application: **24.10.2001**
(21) Application number: 00870075.9
(22) Date of filing: 17.04.2000
(51) Int. Cl.: C07K 14/335, C12N 1/21, A61K 38/16, A61K 35/74, A61P 1/04

(54) **Lactobacillus johnsonii bacteriocin, active against Helicobacter pylori**

(71) Applicant: VRIJE UNIVERSITEIT BRUSSEL, 1050 Brussel (BE)
(72) Inventor: De Vuyst, Luc, 9320 Erembodegem (BE); Avonts, Lazlo, 3052 Oud-Heverlee (BE)
(74) Representative: Van Malderen, Joelle

(57) **Abstract**

The present invention relates to a bacteriocin which shows inhibitory activity against *Helicobacter pylori.* Another aspect of the present invention is a bacteriocin, which is obtainable from *Lactobacillus johnsonii* LMG P-19261.

## Description

### Field of the invention

The present invention relates to a new bacteriocin, the production thereof and the use thereof for the prevention and/or the treatment of bacterial infections, in particular of infections with *Helicobacter pylori.*

### State of the art

Lactic acid bacteria are associated with human food and the environment. Through fermentation, they are responsible for the aroma, flavour and texture development and for the extended shelf-life of many food products. Probiotic lactic acid bacteria display health-promoting properties. Lactic acid bacteria are known to have an antagonistic activity against a variety of microorganisms due to the production of compounds such as organic acids, ethanol, carbon dioxide, free fatty acids, ammonia, diacetyl, hydrogen peroxide, bacteriocin-like compounds, antibiotic-like compounds, and bacteriocins.

Bacteriocin production is one of the properties responsible for the antibacterial activity against closely related Gram-positive species and possibly food spoilers and pathogens. Bacteriocins are natural, antibacterial peptides or proteins, ribosomally produced and excreted by bacteria. Bacteriocins produced by lactic acid bacteria are considered as future food biopreservatives due to their interesting antimicrobial spectrum and their technologically favourable properties. In view of the emerging antibiotic resistance of pathogenic bacteria, they are even considered for the prevention and the treatment of bacterial infections. The development of appropriate fermentation and isolation techniques is necessary for the validation of new bacteriocins produced by lactic acid bacteria.

*Lactobacillus johnsonii* LA1 (formally known as *Lactobacillus acidophilus* LA1) is a commercial probiotic organism. It has been shown that this strain, *in vitro* as well as *in vivo*, is active against Gram-positive and Gram-negative pathogenic bacteria, such as *Staphylococcus aureus, Listeria monocytogenes*, *Salmonella typhimurium*, *Shigella flexneri*, *Klebsiella pneumoniae*, *Pseudomonas aeruginosa* and *Enkerobacter cloacae.* No activity can be shown against the normal intestinal flora such as *Lactobacilli* and *Bifidobacteria*.

Several protocols and chromatographic methods have been proposed for the analytical purification to homogeneity of lactic acid bacteria bacteriocins. Chromatography is usually applied after a first concentration step by salt precipitation or acid extraction. The procedure further includes cationic exchange, hydrophobic interaction, gel filtration and/or reversed-phase chromatography. However, few simple procedures have been disclosed. Methods describe batch adsorption to an appropriate adsorbent based on hydrophobic or electrostatic interactions, acid extraction and reversed-phase high-performance liquid chromatography (RP-HPLC), ammonium sulphate precipitation and reversed-phase chromatography, pH-mediated cell adsorption-desorption and semi-preparative RP-HPLC, and ethanol precipitation, preparative isoelectric focusing and ultrafiltration.

Recently, it has been shown that a whey based culture supernatant of *Lactobacillus johnsonii* LA1 inhibits the growth of *Helicobacter pylori* (P. Michetti et al., Digestion 1999, 60, pp. 203-209). However, the agent responsible for the inhibition of the growth of *Helicobacter pylori* has not yet been identified.

Lactic acid bacteria are known to compete with other microorganisms on mucosal surfaces, a process named "bacterial interference". In order to colonise a mucosal, epithelial surface and to interfere with pathogens *in vivo*, *Lactobacilli* must present certain properties including adhesion, competitive exclusion capacity, and production of inhibitors. However, very few strains have a capacity to adhere and even less to reside in a stomach as this is a hostile environment due to low pH. An important exception is *Helicobacter pylori,* which will usually persist in an infected host for a lifetime. *Helicobacter pylori* causes chronic gastritis, is important in the development of peptic ulcers, and is a risk factor in the development of gastric carcinomas and gastric lymphomas. Current treatments of infection with *Helicobacter pylori* are based on antibiotics associated with antisecretory agents. As the resistance to antibiotics of microbial strains is evolving quickly to the situation that the use of antibiotics on a large scale is rendered ineffective in the near future, there is a need for a simple and inexpensive alternative. Further, prevention or limitation of infection with *H. pylori* in an easy and/or inexpensive way are possible alternatives for the treatment with antibiotics, when started at an early stage e.g. when used in a probiotic such as the commercially available probiotics preparation.

### Aims of the invention

A first aim of the present invention is to provide a novel bacteriocin, possibly comprised in a functional product such as a probiotic.

A further aim of the present invention is to provide a method for the production and/or purification of said bacteriocin.

A second aim of the present invention is to provide an alternative treatment and/or prevention method against *Helicobacter pylori* infection.

### Summary of the invention

A first aspect of the present invention is a bacteriocin characterised in that it shows inhibitory activity against *Helicobacter pylori.* Said bacteriocin can be obtainable from *Lactobacillus johnsonii* LMG P-19261. Said bacteriocin is preferably obtainable from *Lactobacillus johnsonii* LMG P-19261 by the following steps:
- Obtaining a crude cell-free culture supernatant from a fermentation culture comprising *Lackobacillus johnsonii* LMG P-19261,
- A purification step to obtain an at least partially purified bacteriocin

Said purification step preferably comprises an ammonium sulphate treatment step, a recovery step to obtain a surface pellicle, and resuspension of the surface pellicle.

The purification step can further comprise an organic solvent extraction/precipitation step. Preferably, chloroform/methanol is used for this step, but other possibilities exist.

The bacteriocin can be further characterised in that it is active at neutral pH and/or in that it can withstand a heat treatment of at least 30 minutes at 100°C without substantial inactivation.

A second aspect of the present invention is a bacteriocin, characterised that it is obtainable from *Lactobacillus johnsonii* LMG P-19261. Said bacteriocin is preferably obtainable from *Lactobacillus johnsonii* LMG P-19261 by the following steps:
- Obtaining a crude cell-free culture supernatant from a fermentation culture comprising *Lactobacillus johnsonii* LMG P-19261,
- A purification step to obtain an at least partially purified bacteriocin

Said purification step preferably comprises an ammonium sulphate treatment step, a recovery step to obtain a surface pellicle, and resuspension of the surface pellicle.

The purification step can further comprise an organic solvent extraction/precipitation step. Preferably, chloroform/methanol is used for this step, but other possibilities exist.

The bacteriocin can be further characterised in that it is active at neutral pH and/or in that it can withstand a heat treatment of at least 30 minutes at 100°C without substantial inactivation.

Another aspect of the present invention is a micro-organism other than *Lactobacillus johnsonii* LMG P-19261 comprising the bacteriocin of the present invention.

Another aspect of the present invention is a micro-organism genetically engineered to produce a bacteriocin of the present invention. Said micro-organism can be derived from than *Lactobacillus johnsonii* LMG P-19261 or can be a different micro-organism. Said micro-organism also be genetically engineered to provide a higher production or better excretion of the bacteriocin of the present invention.

Another aspect of the present invention is a probiotic composition comprising a bacteriocin according to the present invention and a nutritionally and/or pharmaceutically acceptable carrier.

The probiotic composition can further comprises a micro-organism, preferably a micro-organism according to the present invention as disclosed higher.

Another aspect of the present invention is a bacteriocin or a probiotic composition of the present invention for use as a probiotic.

Another aspect of the present invention is a bacteriocin or a probiotic composition of the present invention for use as a medicament.

Another aspect of the present invention is a bacteriocin as described higher or a probiotic composition of the present invention for the prevention of infection with *Helicobacter pylori.*

Another aspect of the present invention is a bacteriocin or a probiotic composition of the present invention for curing an infection with *Helicobacter pylori.*

Another aspect of the present invention is the use of the bacteriocin or of a probiotic composition according to the present invention for the manufacture of a medicament for the probiotic treatment of an animal, including a human.

Another aspect of the present invention is the use of the bacteriocin or of a probiotic composition according to the present invention for the preparation of a medicament for the prevention or inhibition of an infection with *Helicobacter pylori* in an animal, including a human.

### Short description of the drawings

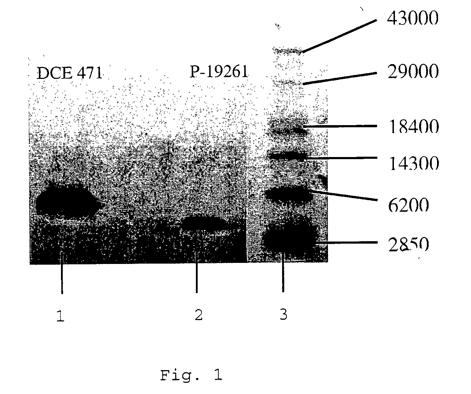

Figure 1 describes the detection of antibacterial activity as visualised on a polyacrylamide gel.

### Detailed description of the invention

A deposit has been made according to the Budapest Treaty for the micro-organism *Lactobacillus johnsonii IMDO214001* under deposit number LMG P-19261 at the BCCM/LMG Culture Collection, Laboratorium voor Microbiologie, Ledeganckstraat 35, B-9000 Gent (Belgium)

### Example 1: Bacteriocin production and isolation from Lactobacillus johnsonii LMG P-19261

For isolation of bacteriocin, crude culture supernatants were obtained from flask cultures (2 liters; 37°C) harvested after 12-13 h of fermentation. The cells were removed by centrifugation (5500 g, 30 min, 4°C) and the supernatant fraction was adjusted to pH 6.5 (with 10 N NaOH).

### Assay of bacteriocin activity and inhibitory spectrum

Bacteriocin activity was measured by an agar spot test as described previously (De Vuyst *et al.,* 1996). Briefly, twofold dilutions of cell-free culture supernatant or partially purified bacteriocin (see below) were spotted (10 *µ*l) onto fresh indicator lawns of *Lactobacillus delbrueckii* subsp. *bulgaricus* LMG 6901^{T} as indicator organism. These lawns were prepared by propagating fresh cultures to an optical density at 600 nm (OD₆₀₀) of 0.40-0.45 and adding 100 *µ*l of the cell suspension to 3.5 ml of overlay agar (0.7 % agar). Overlaid agar plates were incubated for at least 24 h at 37°C. Activity was defined as the reciprocal of the highest dilution demonstrating complete inhibition of the indicator lawn and was expressed in activity units (AU) per ml culture medium.

To assay the spectrum of activity, the antimicrobial activity of cell-free culture supernatant, and of concentrated preparations of ammonium sulphate precipitate (see below) and partially purified bacteriocin (see below) was examined against a wide range of target bacteria (Gram-positives) in at least two separate tests.

The positive results are presented in Table 1.

The partially purified bacteriocin preparation displayed bactericidal activity towards different lactobacilli and enterococci. None of the *Lactococcus*, *Leuconostoc*, *Pediococcus*, *Streptococcus,* and *Bifidobacterium* strains tested were inhibited.

**Table 1.**

| Activity (AU/ml) of cell-free culture supernatant (Sup), ammonium sulphate precipitate (surface pellicle) (ASP) and chloroform/methanol extract (C/M) derived from a culture of *Lactobacillus johnsonii* LMG P-19261 towards Gram-positive bacteria - n.d. = not determined | | | |
|---|---|---|---|
| Lb. john sonii LMG P-19261 | | | |
| ***Indicator*** | sup | ASP | C/M |
| *E. casseliflavus* LMG 10745 | 0 | 800 | 150 |
| E. *faecalis* LMG 16216 | 0 | 50 | 0 |
| *E. faecalis* LMG 16337 | 0 | 50 | 0 |
| *E. faecalis* LMG 8222 | 0 | 50 | 50 |
| *E. faecalis* V24 | 0 | 50 | 0 |
| *E. gallinarum* LMG 13129 | 0 | 50 | 50 |
| *Lb*. *acidophilus* LMG 13550 | 0 | 0 | 200 |
| *Lb. delbrueckii* subsp. | 0 | 800 | 600 |
| *bulgaricus* LMG 13551 | | | |
| *Lb*. *delbrueckii* subsp. | 50 | 4800 | 4800 |
| *bulgaricus* LMG 6091^{T} | | | |
| *Lb*. *fermentum* LMG 13554 | 0 | 100 | 100 |
| *Lb*. *helveticus* 102 | 100 | 1600 | 3200 |
| *Lb*. *helveticus* LMG 11445 | 50 | 6400 | 3200 |
| *Lb*. *helveticus* LMG 13555 | 0 | 3200 | 400 |
| *Lb*. *sakei* LMG 13558 | 0 | 1600 | 1200 |
| *Lb*. *amylovorus* DCE 471 | n.d. | n.d. | 50 |
| *Lb*. *amylovorus* LMG 13135 | n.d. | n.d. | 100 |
| *Lb*. *amylovorus* LMG 13049 | n.d. | n.d. | 150 |
| *Lb*. *amylovorus* LMG 9496^{T} | n.d. | n.d. | 800 |
| *Lb*. *amylovorus* LMG 9434 | n.d. | n.d. | 600 |
| *Lb*. *amylovorus* LMG P-13139 | n.d. | n.d. | 600 |

### Partial purification of the antibacterial peptide from Lactobaclllus johnsonii LMG P-19261

A partially purified bacteriocin preparation was prepared as described by De Vuyst et al. (1996). Briefly, cells were harvested from 2-litre cultures by centrifugation (5500 g, 30 min, 4°C) after 12-13 h of fermentation at 37°C when the pH had dropped to approximately 4.1. The supernatant fraction was adjusted to pH 6.5 (with 10 N NaOH). This material was referred to as crude supernatant.

Crude cell-free culture supernatant (pH 6.5) was made up to 40 % ammonium sulphate saturation and held overnight at 4°C with stirring. The sample was centrifuged (5500 g, 30 min, 4°C) and the surface pellicle (containing the bacteriocin) was recovered and suspended in 50 mM sodium phosphate buffer (pH 6.5). This material was considered as crude bacteriocin.

Crude bacteriocin was further treated with at least 25 volumes of a mixture of chloroform and methanol (2 :1, v/v) for 1 h at 4°C. After sedimentation, the clear supernatant was removed and another 15 volumes of a mixture of chlorform and methanol (2 :1, v/v) were added followed by incubation during 1 h at 4°C. The resulting fine-grained white precipitate (containing the bacteriocin) was collected by centrifugation for 30 min at 5500 g (4°C), air-dried and dissolved in a minimal amount of ultrapure water. It was considered as partially purified bacteriocin and could be stored at -80°C.

The results of the purification are presented in Table 2.

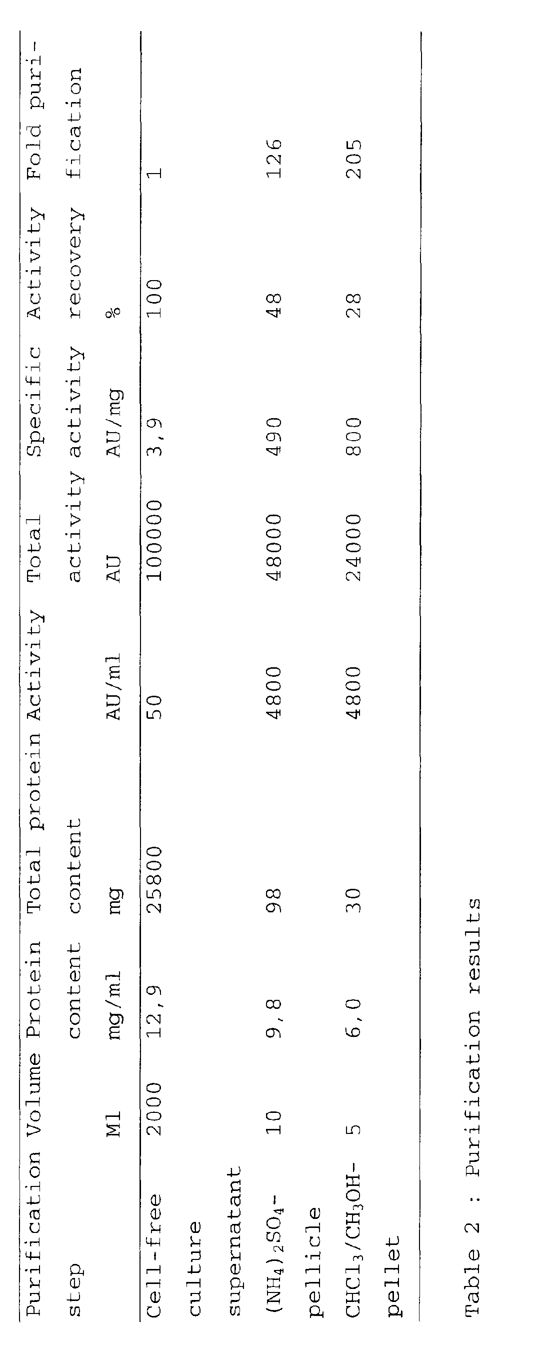

### Characterization of the antibacterial peptide from Lactobacillus johnsonii LMG P-19261

For a characterization of the antibacterial substance, two volumes of a bacteriocin preparation were treated during 2 h with one volume of the following enzymes (final concentration, buffer and incubation temperature between brackets) and tested for activity as described above: trypsine (2 mg/ml, 50 mM sodium phosphate buffer pH 7.0, 37°C); proteinase K (1 mg/ml, 50 mM sodium phosphate buffer pH 7.5, 37°C); alpha-chymotrypsine (5 mg/ml, 50 mM sodium phosphate buffer pH 7.5, 37°C); alpha-amylase (1 mg/ml, 50 mM sodium phosphate buffer pH 7.0 + 10 mM NaCl, 25°C); thermostable alpha-amylase (1 mg/ml, 50 mM sodium phosphate buffer pH 7.0 + 10 mM NaCl, incubation at 25°C after a 10 min treatment at 70°C); lysozyme (1 mg/ml, 50 mM sodium phosphate buffer pH 7.0 + 10 mM NaCl, 25°C); lipase (1 mg/ml, 50 mM sodium phosphate buffer pH 7.2, 37°C), pepsine (1 mg/ml, 200 mM citrate buffer pH 2.2, 37°C); and catalase (433.333 U/ml, ultrapure water pH 7.0, 25°C). As positive control only buffer was added.

Heat stability was tested by treating partially purified bacteriocin (6400 AU/ml) during 15 min, 30 min and 60 min at 60°C and 100°C and during 15 min at 121°C, after which the bioactivity was tested quantitatively.

pH stability was tested by incubation of a partially purified bacteriocin solution (4800 AU/ml) in different buffers: 200 mM citrate buffer of pH 2.2, 3.0, 4.0, 4.5, 5.0, 5.5 and 6.0, and 100 mM sodium phosphate buffer of pH 6.5, 7.0 and 8.0 The bioactivity was tested after 1 h and after 24 h of incubation at 25°C.

The results are presented in Table 3, 4 and 5.

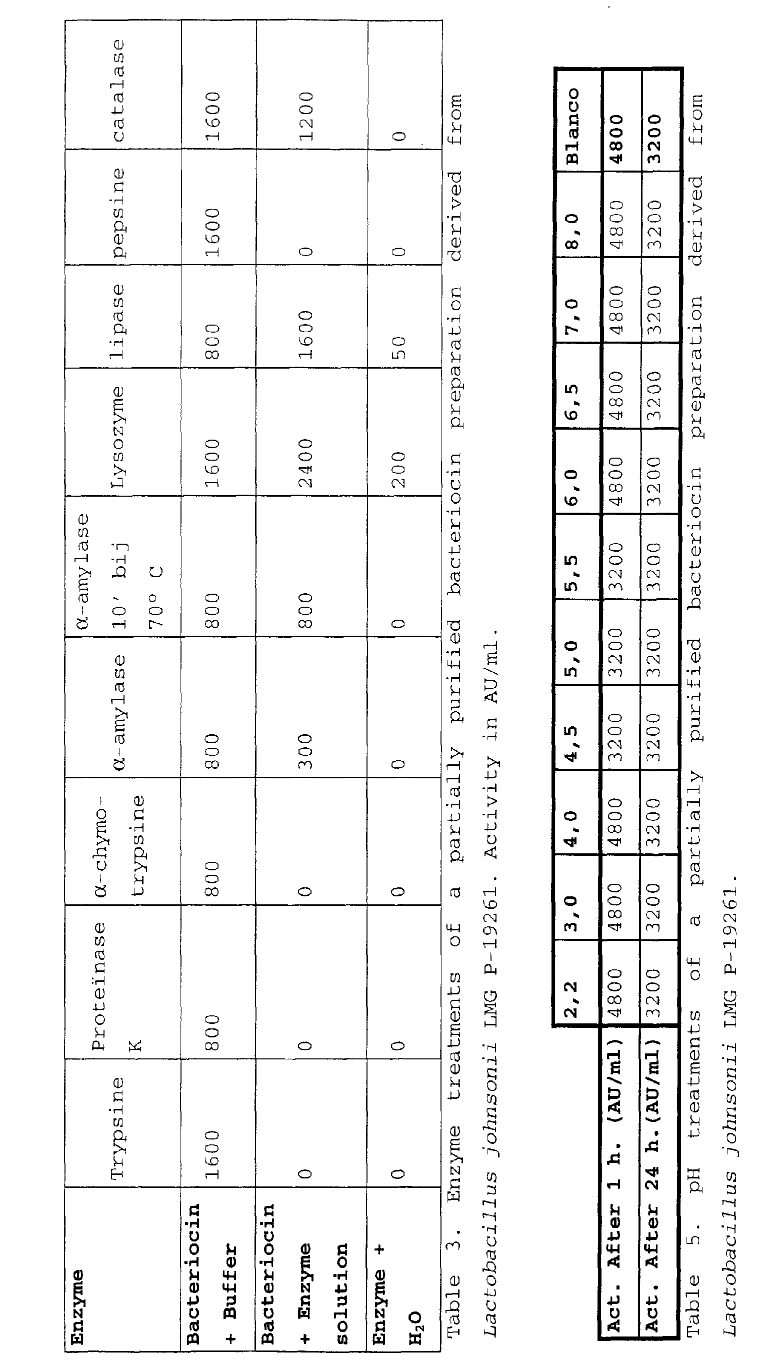

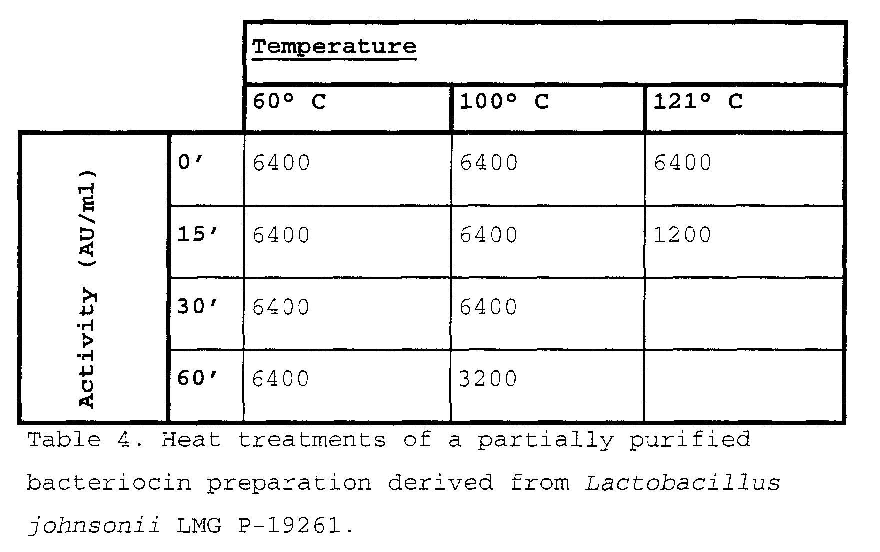

The antagonistic compound produced by *Lb. johnsonii* LMG P-19261 was characterised as a proteinaceous substance (antibacterial peptide), since it was completely degraded by proteolytic enzymes such as trypsine, proteinase K, alpha-chymotrypsine, and pepsine. Thermostable alpha-amylase (proteinase-free), lipase, lysozyme and catalase had no effect. It was further precipitable with ammonium sulphate (see above) and it retained its activity at pH 6.5 (adjusted cell-free culture supernatant), eliminating the possible inhibition due to undissociated lactic acid. The antibacterial peptide was extremely heat-stable (at least 30 min at 100°C, and only partially inactivated after a treatment of 60 min at 100°C or 15 min at 121°C). Finally, the antibacterial peptide was active and stable in a wide pH range. Hence, it displayed the general characteristics of a bacteriocin produced by lactic acid bacteria (De Vuyst & Vandamme, 1994).

### Estimation of the molecular mass by tricine-SDS-PAGE

Tricine-sodium dodecyl sulphate-polyacrylamide gel electrophoresis (tricine-SDS-PAGE) was carried out as described by Schägger & von Jagow (1987). Polyacrylamide concentrations in the stacking gel and separating gel were 9.6 and 16.0 %, respectively. Electrophoresis was conducted at a constant voltage of 30 V for at least 1 h followed by 90 V for at least 12 h. After extensive washing during 5 h with regularly replaced sterile ultrapure water, the gel was transferred to a MRS-agar plate and overlayed with soft agar (0.7 %) inoculated with a culture (OD₆₀₀ of 0.45) of the indicator organism *Lb. delbrueckii* subsp. *bulgaricus* LMG 6901^{T}.

The result is presented in Figure 1.

Figure 1 describes the detection of antibacterial activity as visualised on a polyacrylamide gel. After tricine-sodium dodecyl sulphate-polyacrylamide gel electrophoresis, the gel was placed on a MRS agar plate and overlayed with MRS soft agar containing *Lactobacillus delbrueckii* subsp. *bulgaricus* LMG 6901^{T} as indicator organism. Lane 1, partially purified amylovorin L471 derived from *Lactobacillus amylovorus* DCE 471; lane 2, partially purified bacteriocin derived from *Lactobacillus johnsonii* LMG P-19261 ; lane 3, molecular mass protein standards.

After tricine-SDS-PAGE and overlaying the gel with a sensitive indicator organism, a protein band showed inhibitory activity. The molecular mass of the antibacterial peptide from *Lb*. *johnsonii* LMG P-19261 was estimated at between 3000 and 4000 Da. The molecular mass of amylovorin L471 from *Lb*. *amylovorus* DCE 471 is estimated at around 4800 Da. The molecular mass of amylovorin L471 as determined by electrospray mass spectrometry is 4877 Da (Callewaert *et al.,* 1999).

### Example 2: Inhibition test towards Helicobacter pylori strains

*Helicobacter pylori* strains were cultivated on Muller-Hinton agar medium (Oxoid). Partially purified bacteriocin preparation was spotted on these plates. The plates were incubated anaerobically (10 % CO₂, 10 % H₂ and 80 % N₂) for three days and checked for zones of inhibition. Bacteriocin preparations were also treated with one volume of proteinase K (final concentration of 1 mg/ml) and alpha-chymotrypsine (final concentration of 5 mg/ml) solution. The enzymes were dissolved in 50 mM sodium phosphate buffer pH 7.5. As positive control only buffer without enzyme was added. The sensitive *Lb*. *delbrueckii* subsp. *bulgaricus* LMG 6901^{T} strain was also used as a control.

The results are presented in Table 6.

**Table 6.**

| Inhibition of *Helicobacter pylori* strains by a partially purified bacteriocin preparation of *Lactobacillus johnsonii* LMG P-19261. The latter had an activity of approximately 6400 AU/ml against *Lactobacillus delbrueckii* subsp. *bulgaricus* LMG 6901^{T}. + = inhibition; - = no inhibition. | | | |
|---|---|---|---|
| Indicator strain | Partially purified bacteriocin | Proteinase K treatment | Alphachymotrypsin treatment |
| *H*. *pylori* 1 | + | +/- | +/- |
| *H. pylori* 2 | - | - | - |
| *Lb. delbrueckii* subsp. | + | - | - |
| *bulgaricus* LMG 6901^{T} | | | |

### Detection of the lactobin A (= amylovorin L471) structural gene in Lactobacillus johnsonii LMG P-19261

Total genomic DNA was isolated by use of the Puregene kit (Pharmacia Biotech). DNA was checked on a 2 % (m/v) agarose gel and quantified by the Dynaquant kit (Pharmacia) with calf thymus DNA as standard (DQ 202, Sigma). To detect the lactobin A structural gene, three primers were chosen from the DNA sequence of the coding part of the lactobin A structural gene (Fig. 2). The first primer (5' TGG ACT AAT GCA TAC AGC GC 3'), named LBN sense primer, corresponded to the N-terminal end of lactobin A (Contreras *et al.,* 1997). The second primer (5' CCA ATT ACA GCA CCC CAT AC 3'), named LBN antisense primer, corresponded to the C-terminal part of lactobin A (Contreras *et al.,* 1997). The third primer (5' CTT TAC GAA CAT AAC CCG CC 3'), named AMY antisense primer, corresponded to the C-terminal part of the known N-terminal amylovorin L471 amino acid sequence, which is identical to the N-terminal lactobin A amino acid sequence (Callewaert *et al.,* 1999). Two PCR experiments were performed, one with primers 1 and 2 (PCR I) and another with primers 1 and 3 (PCR II). Total DNA from *Lb*. *amylovorus* LMG P-13139 (producer of lactobin A) and *Enkerococcus faecium* CTC 492 (not producing lactobin A neither amylovorin L471) were used as positive and negative controls, respectively. A Perkin Elmer GeneAmp PCR System 9600 (Perkin Elmer Biosystems, Vaterstetten, Germany) was used. The PCR reaction was done in a volume of 50 *µ*l Gold PCR buffer (Perkin Elmer Biosystems) containing 2.5 mM of MgCl₂, 0.2 mM of dNTP, 0.6 *µ*M of each primer, 1.25 U of AmpliTaq Gold DNA polymerase (Perkin Elmer Biosystems) and 5 ng of template DNA. The amplification was started by a DNA denaturation and an enzyme activation step of 10 min at 95°C. The amplification consisted of 35 cycles of denaturation and enzyme activation during 30 s at 95°C and annealing and extension during 30 s at 67°C. Final annealing and extension occurred during 12 min at 67°C. At the end the temperature was decreased to 4°C to stop the reaction. PCR amplification was optimised with respect to temperature and quantity of template DNA (results not shown). Amplified DNA fragments were visualised on a 4.5 % (m/v) LSI MP agarose gel (Boehringer Mannheim) after staining with ethidium bromide. A φX174/*Hin*fI DNA marker (726 to 24 bp ; Stratagene, CA, USA) was used to determine the length of the amplified fragments. The gel was run at 150 V for 150 min. Cleaning of the PCR products to be sequenced was done by use of the QIA quick spin PCR purification kit (QIAGEN, Hilden, Germany). The sequencing reaction was carried out with 4 *µ*l Big Dye (Perkin Elmer Biosystems), 3 *µ*l PCR product, and 3 *µ*l primer (LBN sense and LBN antisense or AMY antisense primer) of 20 ng/*µ*l. The sequence of double-stranded DNA in two orientations was determined on a Perkin Elmer Biosystems ABI PRISM 377 DNA Sequencer (Perkin Elmer Biosystems).

With the positive control strain *Lb*. *amylovorus* LMG P-13139, two amplicons were detected in total DNA: a 144 bp fragment and a 96 bp fragment corresponding with the amplification with the LBN sense and LBN antisense primer set (PCR I), and the LBN sense and AMY antisense primer set (PCR II), respectively. A very weak signal was observed with *Lb*. *johnsonii* LMG P-19261, indicating that possibly a homologous bacteriocin gene is present. Extensive PCR amplification followed by sequence analysis revealed the same nucleotide sequence as lactobin A, at least between the primers used.

### References

Callewaert, R., Holo, H., Devreese, B., Van Beeumen, J., Nes, I. & De Vuyst, L. (1999). Characterization and production of amylovorin L471, a bacteriocin purified from *Lactobacillus amylovorus* DCE 471 by a three-step method. *Microbiology* **145**, 2559-2568.

Contreras, B.G.L., De Vuyst, L., Devreese, B., Busanyova, K., Raymaeckers, J., Bosman, F., Sablon, E. & Vandamme, E.J. (1997). Isolation, purification, and amino acid sequence of lactobin A, one of the two bacteriocins produced by *Lactobacillus amylovorus* LMG P-13139. *Applied and Environmental Microbiology* **63**, 13-20.

De Vuyst, L., Callewaert, R. & Pot, B. (1996). Characterization of the antagonistic activity of *Lactobacillus amylovorus* DCE 471 and large scale isolation of its bacteriocin amylovorin L471. *Systematic and Applied Microbiology* **19**, 9-20.

De Vuyst, L. & Vandamme, E. J. (Eds.). (1994). *Bacteriocins of Lactic Acid Bacteria: Microbiology, Genetics and Applications*. Blackie Academic & Professional, London, 539 pp.

Schägger, H. & von Jagow, G. (1987). Tricine-sodium dodecyl sulphate-polyacrylamide gel electrophoresis for the separation of proteins in the range from 1 to 100 kDa. *Analytical Biochemistry* **166**, 368-379.

## Claims

1. A bacteriocin **characterised in that** it shows inhibitory activity against *Helicobacter pylori.*

2. A bacteriocin, **characterised tha**t it is obtainable from *Lactobacillus johnsonii* LMG P-19261.

3. A bacteriocin such as in claim 2, **characterised in that** it is obtainable from *Lactobacillus johnsonii* LMG P-19261 by the following steps:
• Obtaining a crude cell-free culture supernatant from a fermentation culture comprising *Lactobacillus johnsonii* LMG P-19261,
• A purification step to obtain an at least partially purified bacteriocin

4. A bacteriocin as in claim 3, **characterised in that** the purification step comprises an ammonium sulphate treatment step, a recovery step to obtain a surface pellicle, and resuspension of the surface pellicle.

5. A bacteriocin as in claim 4, **characterised in that** the purification step further comprises an organic solvent extraction/precipitation step.

6. A bacteriocin as in any of the claims 2 to 5, further **characterised in that** it is active at neutral pH.

7. A bacteriocin as in any of the claims 2 to 6, further **characterised in that** it can withstand a heat treatment of at least 30 minutes at 100°C without substantial inactivation.

8. A bacteriocin as in claim 1, **characterised in that** it consists of a bacteriocin according to any of the claims 2 to 7.

9. A microorganism other than *Lactobacillus johnsonii* LMG P-19261 comprising the bacteriocin of any of the claims 1 to 8.

10. A micro-organism genetically engineered to produce a bacteriocin as in any of the claims 1 to 8.

11. A probiotic composition comprising a bacteriocin such as in any of the claims 1 to 8 and a nutritionally and/or pharmaceutically acceptable carrier.

12. A probiotic composition such as in claim 11, **characterised in that** it further comprises a microorganism.

13. A probiotic composition such as in claim 12, **characterised in that** said micro-organism is a micro-organism as in claim 10.

14. A bacteriocin as in any of the claims 1 to 8 or a probiotic composition as in claim 11 for use as a probiotic.

15. A bacteriocin as in any of the claims 1 to 8 or a probiotic composition as in claim 11 for use as a medicament.

16. A bacteriocin as in any of the claims 1 or 8 or a probiotic composition as in claim 11 or 12 for the prevention of infection with *Helicobacker pylori.*

17. A bacteriocin as in any of the claims 1 or 8 or a probiotic composition as in claim 11 or 12 for curing an infection with *Helicobacter pylori.*

18. Use of the bacteriocin according to any of the claims 1 to 8 or of a probiotic composition as in claim 11 or 12 for the manufacture of a medicament for the probiotic treatment of an animal, including a human.

19. Use of the bacteriocin according to claim 1 or 8 or of a probiotic composition as in claim 11 or 12 for the preparation of a medicament for the prevention or inhibition of an infection with *Helicobacter pylori* in an animal, including a human.


SEQUENCE LISTING

<110> Vrije Universiteit Brussel

<120> NEW BACTERIOCIN, PRODUCTION AND USE THEREOF

<130> P.VUB.10/EP

<140>
<141>

<160> 3

<170> PatentIn Ver. 2.1

<210> 1
<211> 20
<212> DNA
<213> Artificial Sequence

<220>
<223> Description of Artificial Sequence: LBN Sense
      Primer

<400> 1
tggactaatg catacagcgc                                                20

<210> 2
<211> 20
<212> DNA
<213> Artificial Sequence

<220>
<223> Description of Artificial Sequence: LBN Antisense
      Primer

<400> 2
ccaattacag caccccatac                                                20

<210> 3
<211> 20
<212> DNA
<213> Artificial Sequence

<220>
<223> Description of Artificial Sequence: AMY Sense

Primer

<400> 3
ctttacgaac ataacccgcc                                                    20